Europäisches Patentamt

European Patent Office

Office européen des brevets

(19)

(11) Publication number: **0 171 176 B1**

(12) **EUROPEAN PATENT SPECIFICATION**

(45) Date of publication of patent specification: **02.10.91**   (51) Int. Cl.⁵: **A61L 27/00**

(21) Application number: **85304757.9**

(22) Date of filing: **03.07.85**

Divisional application 91200300.1 filed on 03/07/85.

(54) Bone repair using collagen.

(30) Priority: **06.07.84 US 628328**
**06.07.84 US 628335**
**06.07.84 US 628404**
**06.07.84 US 628409**

(43) Date of publication of application:
**12.02.86 Bulletin 86/07**

(45) Publication of the grant of the patent:
**02.10.91 Bulletin 91/40**

(84) Designated Contracting States:
**AT BE CH DE FR GB IT LI LU NL SE**

(56) References cited:
**EP-A- 0 082 621       US-A- 3 458 397**
**US-A- 3 949 073       US-A- 4 394 370**
**US-A- 4 440 750       US-A- 4 455 256**

(73) Proprietor: **COLLAGEN CORPORATION (a Delaware corporation)
2500 Faber Place
Palo Alto California 94303(US)**

(72) Inventor: **Wallace, Donald G.
387 Hedge Road
Menlo Park California 94025(US)**
Inventor: **Piez, Karl A.**
**1120 Bay Laurel Drive
Menlo Park California 94025(US)**
Inventor: **Smestad, Thomas L.
1023 Emerson Street
Palo Alto California 94301(US)**
Inventor: **Seyedin, Saeid
645 Cheshire Way
Sunnyvale California 94087(US)**
Inventor: **McPherson, John M.
764 Sequoia Drive
Sunnyvale California 94086(US)**
Inventor: **Armstrong, Rosa
2106 Louis Road
Palo Alto California 94303(US)**

(74) Representative: **Harrison, David Christopher et al
MEWBURN ELLIS & CO 2/3 Cursitor Street
London EC4A 1BQ(GB)**

## Description

This invention relates to the field of bone repair in vertebrates, especially mammals and humans. More specifically, the invention relates to a method of repairing bone which utilizes a collagen-based implant material to facilitate repair by bone conduction. This collagen material is highly purified and non-immunogenic, and may, if desired, be xenogeneic. The collagen is a reconstituted material from bone derived material, or a mixture thereof with purified atelopeptide fibrillar skin collagen.

The problem of effecting repair of defective bones has plagued mankind for centuries. Until relatively recently, the only practical course was to immobilize broken bones and to rely on nature to effect regrowth of skeletal tissue into an injury. Only with the advent of the possibility of surgery has it been possible to utilize implanted bone substitutes, not only to replace injured or diseased bone structures, but also to repair congenital or degenerative defects in the skeletal structure.

A wide range of materials have since been utilized, and elaborate designs have been disclosed for replacements of entire portions of bones, for example hip joints (U.S. 3,820,167) and teeth (U.S. 4,186,486). Materials employed have included metals such as titanium (EPO publication No. 0071242, published 9 February 1983; U.S. 3,918,100), ceramics such as aluminum oxide (U.S. 3,919,723), shaped and treated bone (U.S. 3,318,774), and various bone preparations such as, for example, bone dust compacted into flexible mats (U.S. 2,621,145).

It has long been understood that skeletal structures have inorganic and organic components. The mineral component which, presumably, accounts for the strength and rigidity of bone structure is predominantly a form of calcium phosphate, hydroxyapatite. The organic component is chiefly composed of a single type of protein, collagen, which serves to impart a measure of resilience thus preventing the structures from being unduly brittle. As skeletal tissue is alive, of course, additional metabolically active organic components must be included in the structure, and it is these bone cells and their active metabolites which are responsible for the naturally occurring healing and maintenance processes.

However, since the major components of bone from a quantitative standpoint are collagen and ceramic, various reconstituted implant preparations involving mixtures of similar or different ceramic materials and various types of collagen preparations have also been employed. For example, see U.S. 3,443,261, Hayashi, K., et al, Arc Orthop Traumat Surg (1982) 99:265; and U.S. 4,314,380).

It has been determined that bone tissue repair occurs by one of two alternative mechanisms, or a combination of both. In conductive repair, cells which are already committed to their character as bone cells (osteoprogenitor cells) move into the space of the defect from adjacent bone, and form bone directly. No special factors (other than non-specific nutrients) are required. In induction, however, this process is preceded by conversion of previously uncommitted multipotential cells into osteoprogenitor cells which first form cartilage that calcifies and degenerates and is replaced by bone. In order to acquire the capacity to do this, specific protein factors are required. The nature of these factors is not at present understood. For either conductive or inductive repair, it is required that the living tissue of the host provides the ultimate skeletal structure. Thus the implant which mediates these processes serves not as a substitute for the defective or removed bone, but rather as a matrix support for active replacement of the missing tissue.

Accordingly, attempts have been made to devise implants for defective skeletal tissue or lesions in bones and teeth which are intended precisely for this purpose. These implants do not attempt to mimic the composition of the missing bone, but rather to serve as a structural support and a guiding matrix for encroaching bone deposits derived ultimately from the adjacent fresh bone. These supports may provide only matrix support functions, i.e., mediate conductive repair, or may, in addition, include factors which stimulate the differentiation of uncommitted cells to osteoprogenitor cells by providing what are currently known as "osteogenesis factors" (OF) or "bone morphogenic proteins" (BMP). Because collagen is already a familiar material to the metabolically viable cells associated with bone growth, attempts have been made to use implants which are composed predominantly of collagen for both inductive and conductive bone repair.

For implants useful in inductive repair, U.S. patent 4,294,753 discloses a process for preparing a bone morphogenic protein. A purification procedure for an OF which is probably not identical to the BMP of the foregoing patent is disclosed in U.S. 4,434,094 issued 28 February 1984. These factors reside naturally in bone, and preparations of demineralized bone particles, which are used in construction of implants, presumably release this factor in operable form. Both purified and unpurified forms of these factors have been used in various implants.

Other workers have disclosed the results of attempts to utilize collagen preparations alone as a matrix for conductive bone repair activities, i.e., preparations which do not contain factors for maturation of progenitor cells into osteogenic cells, and thus mediate conductive repair.

Cucin, R. L., et al, New York State Journal of Medicine (1979) 1856 used atelopeptide collagen from calf skin, which had been gamma irradiated, for rib repair in rabbits and, when supported by gelatin sponge material or with autologous bone dust, to repair skull holes in dogs.

A preparation of collagen, presumably still containing the teleopeptides, and cross-linked by gamma irradiation was employed in filling tooth pulp cavities and as an under the skin "bone replacement" implant as disclosed in, respectively, EPO publication 0012443 published 25 June 1980 and EPO publication 0012959, published 9 July 1980.

In US-A-4394370 and US-A-440750, powders or particles of dimineralized bone are used in compositions or methods for repairing bone.

The present invention provides an implantable collagen preparation which is capable of conducting the ingrowing bone repair tissue from dedicated bone cells into the defect whose repair is desired. Because xenogeneic collagen can be used, large amounts are obtainable and the method can be widely applied. In addition, the invention provides bone repair compositions which offer great versatility in being adaptable to a wide range of stress-bearing requirements.

Fresh bone containing living osteoprogenitor cells is exposed to a bone defect and placed into contact with a preparation of collagen which is a composition derived from bone or both of two sources, bone and skin. The bone-derived collagen powder is prepared from demineralised bone (DMB) (i.e.may use the materials of US-A-4394370 and US-A-440750 as starting material), and consists essentially of Type I collagen having the telopeptides effectively removed. It is obtained by treating DMB with a non-collagenase protease, such as trypsin, which both destroys factors mediating inductive repair and removes the telopeptides. The resultant material is obtained in powder form and will be referred to herein as bone collagen powder (BCP). Treatment of a bone by pepsin (a protease) per se is seen in US-A-3458397 but then what is treated is cancellous, not demineralized, bone.

The skin-derived collagen is chiefly Type I collagen, includes a small amount of Type III and is typically obtained from calf skin. This type of collagen is commercially available under the trademark Zyderm® collagen soft tissue implant (ZCI) for use in removing wrinkles. The collagen preparation typified by ZCI is a reconstituted fibrillar form of atelopeptide collagen.

As is described for example in US-A-3949073, the skin-derived collagen is typically obtained from skin in a process wherein it has been dissociated into solubilized form, sterilized by filtration, and then reconstituted into fibrillar form after removal of the telopeptides.

By varying the ratio of BCP to ZCI in a mixture of these peptides, the physical properties of the repair material can be adjusted to conform to the particular demands of the environment of the defect.

Thus in one aspect, the invention relates to a composition for effecting conductive repair of a bone defect in a mammal which includes a demineralized Type I collagen preparation characterized in that the preparation consists essentially of a collagen powder which is a protease-treated demineralized bone powder free of lipids.

In other aspects, the invention relates to a Type I collagen powder characterized in that it is a demineralized, protease-treated bone powder free of lipids and to a process for preparing a demineralized Type I collagen preparation characterized in that, to prepare a collagen powder which is a protease-treated demineralized bone powder free of lipids, demineralized bone power is treated with at least one protease other than collagenase to remove proteinaceous components.

## Modes of Carrying Out the Invention

### A. Definitions

As used herein, "conductive" repair of bone defects refers to a process for replacing lost bone or for growing desired new bone, which involves the metabolism of previously committed osteoprogenitor cells. These cells are capable of producing cartilage and/or bone without induction by protein factors generally known as osteogenic or morphogenic. The process includes mechanisms whereby osteogenesis is directly effected by the committed cells, but not those wherein there is a requirement for added protein factors to produce committed cells.

"Consisting essentially of" means that the composition so defined derives all or substantially all of its relevant properties from the ingredient(s) as qualified.

It is realized that conversion of undifferentiated cells to osteoprogenitor cells may still be effected by indigenous proteins. However, as herein defined, "conductive" bone repair is mediated by external supply only of supporting matrix, and does not provide an external source of living tissue or of non-indigenous osteogenesis factor.

"Bone defect" refers to a space in the skeletal system in which it is desired that bony tissue be deposited, whether the space is created by injury to the subject, or by a malformation or degeneration of the subject's skeletal system. Such defects may be the result of simple bone breakage, decay of bone tissue because of disease, surgical removal of diseased bone tissue or of unwanted malformations, or of reconstructive or cosmetic surgery.

"Fresh bone" refers to bone in the skeletal system of the subject organism which is in healthy condition and which is treated such as by cutting to expose living tissue. It is found that in the method of the invention contact is required with the defect area by fresh bone to provide the viable cells for regeneration of bone within the defect.

"Preparation of collagen" refers to a composition which contains as its major component some form of collagen protein.

"Xenogeneic" refers to a species which is different from that of the subject being treated.

"Free from impurities" or "purified" refers to those impurities which are normally associated with the collagen or other preparation in its natural state. Thus, collagen prepared from calf skin is free from impurities when other components of calf skin have been substantially removed; that from bone, when other components of bone are eliminated.

"Reconstituted" collagen refers to collagen which has been disassembled into individual triple helicial molecules with or without their telopeptide extensions and brought into solution, and then regrouped into "fibrillar" form. In this form, the fibrils consist of long, thin collagen molecules staggered relative to one another by multiples of about 1/4 of their length. This results in a banded structure which can be further aggregated into fibers.

"Substantially free of cross-linking" refers to collagen which has undergone removal of the telopeptides, so as to result in a preparation lacking the native capacity for cross-link formation. Such preparations remain substantially cross-link free if not deliberately cross-linked by, for example, treating with glutaraldehyde, or subjected to treatment imposing a spurious form of such linkage. For example, treatments often used for sterilizing purposes, such as high temperature and $\gamma$-irradiation have the effect of causing the formation of additional cross-links between helices. In the preparation used in the invention, the skin-derived collagen is sterilized by microfiltration while still in solution and handled under sterile conditions thereafter, thus avoiding the result of unwanted cross-link formation.

"Bone collagen powder" (BCP) refers to a purified atelopeptide preparation of collagen derived from demineralized bone. This preparation has been treated with at least one protease and is free of lipids, i.e. it consists essentially of collagen per se, and does not contain metabolically active proteins. As it originates in bone, it is composed of Type I collagen, and it mimics the native 3-dimensional structure found in bone.

## B. Detailed Description

### B.1 Applications

The bone defects to which the invention applies include any cavity in osseous tissue whose filling is required in order to integrate the skeletal system. Medical or veterinary procedures of bone defect repair which are appropriate as vehicles for the use of the method of the invention include reconstructive surgery, removal of diseased osseous tissue and replacement with overlay or prosthesis, tightening of teeth, replacement of teeth, repair of traumatic injury, and the like. Precise means for applying the collagen preparations of the invention is dependent on the nature of the bone defect and the procedure selected to counteract it, as will be understood by those skilled in the art. However, in general, the compositions of the invention can be made into a paste or gel and molded into the defect by surgically packing the paste into the defect. The collagen preparation may also be injected into the defect when mixed so as to form a thinner suspension. In all cases, however, the defect to be treated must first be cleansed in such a way so as to expose fresh bone surface so that living bone cells are placed in communication with the defect. The fresh bone surface is required in order to provide a source of osteoprogenitor cells needed to synthesize the permanent bone structure.

### B.2 Collagen Preparations

Native collagen consists in large part of a triple helical structure containing repeating triplet sequences composed of glycine linked to two additional amino acids, commonly proline and hydroxyproline; thus, glycine appears in every third position in the chain. In addition, all collagen chains contain regions at each end which do not have the triplet glycine sequence and are thus not helical. These regions are thought to be responsible for the immunogenicity associated with most collagen preparations. Immunogenicity can, in large part, be mitigated by removal of these regions to produce "atelopeptide" collagen. This can be accomplished by digestion with proteolytic enzymes such as trypsin or pepsin. Because of differing specificities of these proteases, the degree of completeness of removal of the telopeptides varies. Thus certain proteases, which effect the most complete removal, are preferred. Included among these is trypsin, which results in removal of substantially all of the telopeptide portions.

The non-helical telopeptide regions are also required to form the cross-links which aid in stability of the fibrillar structure. These regions contain aldehydes capable of cross-linkage to lysine residues. Atelopeptide collagen must be cross-linked artificially, if it is so desired.

While all collagens share the foregoing characteristics, they have been subclassified into approximately ten types depending on the precise amino acid sequence in the individual chains of the triple helix, the carbohydrate content, and the presence or absence of disulfide cross-linking. The most common subtypes are Type I which is present in skin, tendon, and bone, and which is made by fibroblasts, and Type III which is found primarily in skin. Other types reside in specialized membranes or cartilage or at cell surfaces. Types I and III contain similar numbers of amino acids in their helices; however, Type III but not Type I contains two adjacent cysteines at the C-terminal ends of the triple helix which are capable of forming interchain cross-links.

Type I collagen contains one $\alpha2$ (I) and two $\alpha1$(I) chains each of which contains 1014 amino acids in its triplet region; there are several carbohydrate moieties present on each chain. Type III collagen contains only $\alpha1$(III) (3 chains) which contain 1026 residues in their triplet regions. As stated above, the presence in Type III of a pair of adjacent cysteine residues at the carboxy terminal end of the triplet region results in stability of the interchain cross-links. Both collagens contain short non-triplet ends (telopeptides). The reconstituted fibrillar atelopeptide collagen used in this invention contains the atelopeptide forms of both Type I and Type III; the bone collagen powder consists of the atelopeptide form of Type I exclusively.

The Skin-Derived Collagen

The atelopeptide reconstituted fibrillar skin collagen preparations which may be used in the compositions of this invention are typified by the purified calfskin-derived atelopeptide collagen reconstituted fibrillar suspensions sold commonly under the trademark ZYDERM® collagen implant (ZCI).

This and other preparations of skin-derived purified atelopeptide, reconstituted fibrillar collagen are well known in art. ZCI has been used extensively in soft tissue applications, including most prominently, the removal of wrinkles and depressed scars by injection of the preparation just under the skin. However, such preparations have not been used for bone repair except in conjunction with supporting materials which also contain OF and thus are directed to inductive bone repair. Such applications are disclosed in U.S. Serial No. 348,414, filed 12 February 1982.

As this collagen preparation is derived from calf skin, it contains mainly Type I collagen with approximately 1-5% Type III. The aletopeptide collagen is sterilized while still in solution by suitable filtration techniques and thus is not degraded or cross-linked. It is reconstituted into fibrillar form and packaged under sterile conditions.

Bone-Derived Collagen

The bone collagen powder (BCP) used herein is derived from dimineralized bone, and its collagen component is, accordingly, Type I collagen exclusively.

This collagen preparation is a new material not identical in physical or chemical characteristics to previously prepared forms of collagen and is conveniently stored as a dried powder. In general, bone, for example, bovine, porcine, or other mammalian bone, preferably compact bone, is cleaned, frozen, pulverized and demineralized in hydrochloric acid, or other suitable acid, using standard techniques. The residual organic matter is separated and digested using proteolytic enzymes sequentially or in combination. Neutral proteases, e.g., trypsin, which allow for the selective degradation of non-collagenous proteins are preferred as described by Oliver and Grant in British application GB 1565340A. Certain acid proteases such as pepsin cause partial digestion of the collagen, and are not preferred. Non-proteinaceous bone components are also removed using suitable enzymes such as chondroitinase, hyaluronidase, and various nucleases. Of course, the use of collagenase is inappropriate. After treatment with the appropriate enzymes and treatment to remove any lipids, the insoluble material consists of purified atelopeptide collagen in the form of BCP. It may be used after sterilization by known methods such as gamma irradiation or heat. The cross-linking of this type of collagen is often desirable since any residual antigenic effects of any telopeptide portions remaining after trypsin treatment are thus mitigated.

The resulting BCP is novel. It consists essentially of Type I collagen which apparently retains the original molecular architecture of the bone collagen and is free of the telopeptides.

The BCP is resuspended in physiological buffer for use in compacting into a properly prepared bone defect.

## Mixtures

If mixtures are used to form the implants or paste repair material of the invention, these are obtained using suspensions of reconstituted fibrillar atelopeptide collagen, such as ZCI, and BCP in a range of proportions depending on the physical properties needed to handle the material and to cope with the degree of stress expected to be imparted to the repaired region. Increased amounts of BCP permit additional weight-bearing and stress-tolerating properties to be built into the composition, whereas decreasing its proportion relative to ZCI results in greater flexibility and ease of application. Thus, the method of the invention offers greater versatility in adapting the nature of the repair to the subject defect than do the methods known in the art.

The range of proportions is of course large as either component can be used alone. As the preferred mixture is dependent on the nature of the defect, a general, single preferred range cannot be chosen. However, in general, for weight-bearing defects, a preferred range is from about 50% ZCI/50% BCP (by weight) to about 10% ZCI/90% BCP (by weight); for peridontal or superficial defects, a preferred range is from about 90% ZCI/10% BCP to 50% ZCI/50% BCP (by weight). (All of the above ratios assume a 35 mg/ml suspension of ZCI, and the weights referred to are weights of the entire suspension.)

## C. Examples

The following will illustrate but not limit the invention.

## C.1 Preparation of ZCI

Zyderm® collagen implant (available from Collagen Corporation, Palo Alto, CA) was used as the reconstituted fibrillar atelopeptide component. The reconstituted native type collagen fibrils were used at a concentration of about 35 mg/ml. The preparation had been sterilized during processing by filtration and thus was not subjected to degradative procedures such as heat or irradiation.

## C.2 Preparation of BCP (Bovine)

Bovine femurs were manually cleaned of adherent soft tissue, and the articuar ends of the bone removed near the epiphyseal plate to yield primarily compact bone. The bone shafts were cooled with liquid nitrogen (LN$_2$), split axially and the marrow removed. The compact bone pieces were crushed using a jaw crusher at LN$_2$ temperatures into chips 5 mm or less in size. The bone chips were pulverized in a hammermill, again under LN$_2$.

The bone powder was sieved under a stream of running water into two particle size fractions 125 to 250 µm and 250-425 µm. The two particle sizes were processed in parallel but separate paths.

The bone powder was demineralized in 0.5 M HCl (25:1/vol:weight of bone powder) for three hours at room temperature with the acid replaced with fresh acid after 1-1/2 hours. At the end of the demineralization step, the powder was washed three times with water to remove acid soluble materials. The demineralized bone powder was lyophilized preparatory to protease treatment.

The bone powder was digested with trypsin (bone powder 50 mg/ml, trypsin 2 mg/ml in 0.1 M Na$_2$HPO$_4$ with 0.5 mg/ml NaN$_3$, pH 7.8; i.e., trypsin:bone powder = 1:25). The enzyme digestion was carried on at 15°C over a 10-day period. The digestion supernatant was assayed for soluble protein by the biuret method to monitor the course of digestion.

To remove trypsin digestion products, the bone powder was allowed to settle, the supernate was decanted, and 4 M NaCl (10:1/vol:weight) was added to the solid. The bone powder was stirred at room temperature for 1 hr at which time the powder was allowed to settle and the supernatant was decanted. The 4 M NaCl extraction was repeated for a total of three times.

To remove the sodium chloride, the bone powder was washed six times in 10 volumes of water USP water for injection (WFI) with 10 min stirring in an identical manner as described for the NaCl extraction.

The bone powder was then extracted two times with 10 volumes of acetone to remove any lipids present. Following the acetone extraction, the bone powder was washed five times with 10 volumes at WFI, vacuum filtered and lyophilized to yield bone collagen powder (BCP).

The BCP samples were analyzed chemically for lipid, glycosaminoglycan and amino acid composition, immunologically for residual trypsin, bovine red blood cells, and bovine collagen telopeptides (the antigenic terminal ends of collagen), and by electron microscopy for ultrastructure. With increasing trypsin treatment, preferably 4 to 10 days, the BCP became an increasingly pure bovine Type 1 collagen with the telopeptides largely unavailable. There was no residual trypsin. Electron microscopy showed collagen fibrils with the characteristic band pattern and organization of bovine bone. The amino acid composition showed chemically pure collagen.

The BCP's so prepared were of the two particle sizes and varying degrees of trypsin treatment as summarized in Table 1. All samples were sterilized with $\gamma$-irradiation at 1.5 Mrad.

### Table 1
### Summary of BCP Samples

| ID Numbers | Particle Size ($\mu$m) | Days of Trypsinization |
|---|---|---|
| 2Z * | 125 – 250 | 0 |
| 2A | 125 – 250 | 1 |
| 2B | 125 – 250 | 4 |
| 2C | 125 – 250 | 10 |
| 3Z * | 250 – 425 | 0 |
| 3A | 250 – 425 | 1 |
| 3B | 250 – 425 | 4 |
| 3C | 250 – 425 | 10 |

\* Comparative

#### C.3 Preparation of BCP and ZCI Mixture

The bone collagen powders (BCP), designated 2C and 3C in the paragraph above, were mixed in a 55/45% by weight ratio, and the mixture was sterilized by 1.5 mrad $\gamma$-irradiation.

Two batches of BCP/ZCI mixtures were prepared using this sterilized BCP mixture: A mixture of 33% BCP in ZCI was prepared by mixing 0.33 g BCP (dry) with 0.67 g of ZCI (as a 35 mg/ml suspension--i.e., approximately 0.67 g x 0.035 g/ml of solid). A second mixture was prepared by mixing 0.17 g BCP with 0.83 g of ZCI (35 mg/ml suspension). Each of these materials was mixed thoroughly and filled into 1-1/4 cc syringes for use in the procedures below.

#### C.4 Use of BCP in Bone Repair

Rats, 6 weeks old, were anesthetized and 3 x 7 mm defects were created in the left and right parietal bones of the skull. Bone was removed to full thickness of the skull with a dental burr, and the BCP, as prepared above, and wet with sterile saline was packed into the defect. Control defects were left unfilled. BCP samples 2B, 3Z, 3A, 3B and 3C (Table 1) above were tested. Samples were taken and histology done at 2 and 4 weeks after implantation.

Samples 3Z (no trypsin digestion) and 3A (1 day trypsin) showed inflammation with evidence of an antigenic component. Samples 2B, 3B and 3C at 2 weeks showed a multinucleate cell response, digestion of the BCP with a concomitant osteoblast response, and the deposition of new compact bone. At 4 weeks bone formation was extensive throughout the implant with evidence of fusion of new and old bone. Control defects showed only slight bone growth at the cut edges.

The sera of rats receiving BCP sample 3Z contained circulating antibodies to the BCP. The other BCP samples tested did not elicit an antibody response.

These results are summarized in Table 2.

7

## Table 2

## Histology and Serology in

## the Rat Parietal Model at 28 Days

| BCP Sample | Bone Formation | Multinucleate Cells | Macrophages & Lymphocytes | Circulating Antibodies |
|---|---|---|---|---|
| 3Z * | + | ++ | + | + |
| 3A | + | + | + | - |
| 3B | ++ | + | - | - |
| 3C | ++ | + | - | - |
| 2B | ++ | + | - | - |

* Comparative

C.5 Peritoneal Lavage Assay for Immunogenic Response of BCP

BCP samples 2Z, 2A, 2B and 3B, 30 mg suspended in 7 ml phosphate buffered saline (PBS), were injected intraperitoneally into rats. At 1, 3, 14 and 28 days the peritoneum was lavaged with 100 ml PBS and a differential and total cell count was performed as a quantitative measure of inflammation. The BCP implant was examined histologically and sera were taken for measurement of circulating antibodies.

All samples showed a transient neutrophil response at 1 day, probably related to the particulate nature of the material. At 3, 14 and 28 days, the responding cells were predominantly macrophages with extensive digestion of the BCP evident at 28 days by histology of the implant. Very few immunocompetent cells (lymphocytes) were present at any time. Serology showed rats receiving 2Z developed significant antibody titers against the BCP. Rats receiving 2A, 2B, 2C, or 3B did not develop an antibody response.

BCP provides a novel matrix material derived from bone useful in effecting bone repair by a conductive mechanism. A suspension of the bone collagen powder (BCP) can be shaped into bone defects and provides a stress-bearing replacement for lost bone and which supports new bone growth.

C.6 Use of the BCP/ZCl Mixture in Bone Repair

Twenty-four rats, 6 and 8 weeks old, were anesthetized, and 3 x 7 mm defects were created in the left and right parietal bones of the skull. Bone was removed to full thickness of the skull with a dental burr, and the two lots of the BCP/ZCl mixtures, as prepared above, were packed into the defects. Control defects were filled with BCP alone, ZCl alone, or left unfilled. Samples were taken and histology done at 2 and 4 weeks after implantation.

Early bone formation occurred in the defects filled with BCP/ZCl mixtures at 2 weeks post-implantation. However, the mixtures were not always uniform so that the pattern of bone formation varied depending on the predominant type of collagenous implant present (i.e., BCP or ZCl) in a given region of the defect.

By 4 weeks bone formation was extensive in the ZCl component of the mixture and BCP particles were being incorporated into centers of new osteogenic activity. This, coupled with early remodeling, gave the new bone spanning the defects a more uniform appearance than that seen at 14 days. Both implanted control defects (i.e., BCP alone and ZCl alone) showed osteogenic activity characteristic of the patterns observed in previous studies. Non-implanted controls showed only slight bone growth at the cut edges of the defects.

By varying the relative amounts of purified atelopeptide reconstituted fibrillar collagen, which forms a resilient, gel-like suspension, and of bone collagen powder, which offers compressive strength, implants are formed which offer versatility and effectiveness in providing an appropriate matrix for bone repair by conduction.

## Claims

1. A composition for effecting conductive repair of a bone defect in a mammal which includes a demineralized Type I collagen preparation characterized in that the preparation consists essentially of a collagen powder which is a protease-treated demineralized bone powder free of lipids.

2. A composition according to Claim 1 which includes also a purified atelopeptide reconstituted fibrillar skin collagen.

3. Type I collagen powder characterized in that it is a demineralized, protease-treated bone powder free of lipids.

4. A process for preparing a demineralized Type I collagen preparation characterized in that, to prepare a collagen powder which is a protease-treated demineralized bone powder free of lipids, demineralized bone powder is treated with at least one protease other than collagenase to remove proteinaceous components.

5. A process according to Claim 4 wherein the protease is trypsin.

6. A process according to Claim 4 or Claim 5 including obtaining a collagen - containing residue after the treatment with the protease, recovering the residue and extracting it with a solvent to free it from lipids.

7. A process according to Claim 6 wherein the solvent is acetone.

8. A process according to any one of Claims 4 to 7 which includes enzymatically treating the demineralized bone to remove non-proteinaceous bone components.

9. A process according to any one of Claims 4 to 8 including the further step of sterilizing the powder.

## Revendications

1. Composition pour effectuer une réparation par conduction d'un défaut de l'os chez un mammifère qui comprend une préparation de collagène du Type I déminéralisé, caractérisée en ce que la préparation se compose essntiellement d'une poudre de collagène qui est une poudre d'os déminéralisé traitée à la protéase,sans lipides.

2. Composition selon la revendication 1, qui contient également un collagène de peau fibrillaire reconstitué atélopeptidique purifié.

3. Poudre de collagène du Type I, caractérisée en ce que c'est une poudre d'os déminéralisé, traitée à la protéase,sans lipides.

4. Procédé de préparation d'une préparation de collagène du type I déminéralisé, caractérisé en ce que, pour préparer une poudre de collagène qui est une poudre d'os déminéralisé traitée à la protéase sans lipides, on traite une poudre d'os déminéralisé avec au moins une protéase autre que la collagénase pour éliminer les composants protéinés.

5. Procédé selon la revendication 4, où la protéase est la trypsine.

6. Procédé selon la revendication 4 ou la revendication 5, consistant à obtenir un résidu contenant du collagène après traitement avec la protéase, à récupérer le résidu et à l'extraire au moyen d'un solvant pour le libérer des lipides.

7. Procédé selon la revendication 6, où le solvant est l'acétone.

8. Procédé selon l'une quelconque des revendications 4 à 7, qui comprend le traitement enzymatique de l'os déminéralisé pour éliminer les composants non protéinés de l'os.

9.  Procédé selon l'une quelconque des revendications 4 à 8, comprenant l'étape supplémentaire de stériliser la poudre.

**Patentansprüche**

1.  Zusammensetzung zum Bewirken von konduktiver Reparatur eines Knochendefekt in einem Säuger, die ein entmineralisiertes Typ I Kollagenpräparat umfaßt, dadurch gekennzeichnet, daß das Präparat im wesentlichen aus einem Kollagenpulver besteht, das ein von Lipiden freies Protease-behandeltes entmineralisiertes Knochenpulver ist.

2.  Zusammensetzung nach Anspruch 1, die auch ein gereinigtes rekonstituiertes Atelopeptidfibrillärhautkollagen umfaßt.

3.  Typ I Kollagenpulver, dadurch gekennzeichnet, daß es ein von Lipiden freies entmineralisiertes Protease-behandeltes Knochenpulver ist.

4.  Verfahren zur Herstellung eines entmineralisierten Typ I Kollagenpräparats, dadurch gekennzeichnet, daß, um ein Kollagenpulver herzustellen, das ein von Lipiden freies Protease-behandeltes entminieralisiertes Knochenpulver ist, entmineralisiertes Knochenpulver mit zumindest einer anderen Protease als Kollagenase behandelt wird, um eiweißhältige Bestandteile zu entfernen.

5.  Verfahren nach Anspruch 4, bei dem die Protease Trypsin ist.

6.  Verfahren nach Anspruch 4 oder 5, welches das Erhalten eines kollagenhältigen Rückstands nach der Behandlung mit der Protease, die Wiedergewinnung des Rückstands und dessen Extraktion mit einem Lösungsmittel, um es von Lipiden zu befreien, umfaßt.

7.  Verfahren nach Anspruch 6, bei dem das Lösungsmittel Aceton ist.

8.  Verfahren nach einem der Ansprüche 4 bis 7, das die enzymatische Behandlung des entmineralisierten Knochens zur Entfernung von nicht-eiweißhältigen Knochenbestandteilen umfaßt.

9.  Verfahren nach einem der Ansprüche 4 bis 8, das den weiteren Schritt der Sterilisation des Pulvers umfaßt.